# EUROPEAN PATENT SPECIFICATION

(11) **EP 2 125 821 B1**
(45) Date of publication and mention of the grant of the patent: **13.07.2011**
(21) Application number: 07857066.0
(22) Date of filing: 21.12.2007
(51) Int. Cl.: C07D 487/04

(54) **PROCESS FOR THE PREPARATION OF SILDENAFIL**
VERFAHREN ZUR HERSTELLUNG VON SILDENAFIL
PROCÉDÉ DE PRÉPARATION DE SILDÉNAFIL

(30) Priority: 21.12.2006 WO PCT/CN2006/003530
(43) Date of publication of application: 02.12.2009
(73) Proprietor: Krka Tovarna Zdravil, D.D., Novo Mesto, 8501 Novo Mesto (SI)
(72) Inventor: TIAN, Guanghui, Shanghai 201209 (CN); ZHU, Yi, Shanghai 201209 (CN); LIU, Zheng, Shanghai 201209 (CN); WANG, Zhen, Shanghai 201209 (CN); SHEN, Jingsham, Shanghai (CH); BOMBEK, Sergeja, 8000 Novo Mesto (SI); STROPNIK, Tadej, 1000 Ljubljana (SI)
(74) Representative: UEXKÜLL & STOLBERG
(86) International application number: PCT/EP2007/011355
(87) International publication number: WO 2008/074512

(56) References cited:
- EP-A- 0 812 845
- WO-A-01/19827
- WO-A-02/074774
- JOSHI, Y. C. ET AL: "Synthesis of some new dialkyl/diaryl 1,3-diketones and 1,3-diketoesters of 5-(2-ethoxyphenyl)-1-methyl-7-chloro-1H-py razolo[4,3-d]pyrimidines" INDIAN JOURNAL OF HETEROCYCLIC CHEMISTRY , 16(1), 81-82 CODEN: IJCHEI; ISSN: 0971-1627, 2006, XP008090300
- KUMAR, RAJESH ET AL: "Synthesis, spectral studies and biological activity of 3H-1,5-benzodiazepine derivatives" ARKIVOC (GAINESVILLE, FL, UNITED STATES), [Online] vol. 13, 2007, pages 142-149, XP002476137 Retrieved from the Internet: URL:HTTP://CONTENT.ARKAT-USA.ORG/ARKIVOC/J OURNAL_CONTENT/MANUSCRIPTS/2007 /07-2486BP%20AS%20PUBLISHED%20MAINMANUSCRI PT.PDF> [retrieved on 2008-04-11]

## Description

The present invention relates to a process for the preparation of sildenafil and pharmaceutically acceptable salts and solvates thereof. The invention is also directed to a process for the preparation of a pharmaceutical composition comprising sildenafil or a pharmaceutically acceptable salt or solvate thereof. Moreover, the invention relates to intermediates suitable for use in the above processes as well as processes for their preparation.

### BACKGROUND OF THE INVENTION

The compound of formula **(I):** 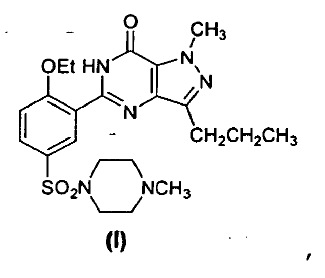 having the chemical name 5-[2-ethoxy-5-(4-methylpiperazin-1-ylsulfonyl)phenyl]-1-methyl-3-n-propyl-1,6-dihydro-7*H-*pyrazolo[4,3-d]pyrimidin-7-one, is also known by its generic name sildenafil. The compound was originally used in the treatment of cardiovascular diseases, such as angina, hypertension, heart failure, atherosclerosis, etc. Later it was found that this compound is particularly useful in the treatment of male erectile dysfunction disease.

Sildenafil is a selective phosphodiesterase type 5 inhibitor. This compound and its preparation were originally disclosed in EP-A-0 463 756 (corresponding to CN1057464A), and it has been found useful in the treatment of certain cardiovascular diseases. Its use in the treatment of male erectile dysfunction was first disclosed in WO-A-94/28902 (corresponding to CN1124926A). An improved process for the preparation of sildenafil is described in EP-A-0 812 845 (corresponding to CN1168376A). CN1208337C discloses a process for the preparation of sildenafil by an inorganic oxidant. CN1176081C and CN1281851A describe two processes for the preparation of sildenafil and its intermediates. Bioorg. Med. Chem. Lett. 2000, 10, 1983-1986 describes a convergent process using polymer-supported reagents in a multi-step reaction, resulting in a clean and efficient preparation without the need for conventional purification methods. US-B-6 204 383 discloses a process using a less basic intermediate in the preparation of sildenafil. WO-A-2001/019827 discloses a process for the preparation of sildenafil in a cost-efficient manner by methylation.

Most of the processes for the preparation of sildenafil in the prior art give rise to side-reactions. Therefore, the yield of the final product is reduced and utility of these prior art processes in pharmaceutical industry is limited.

### DESCRIPTION OF THE INVENTION

The present invention provides a novel process for the preparation of sildenafil and its intermediates, which process has many advantages over prior art processes such as reducing side reactions and improving the yield of the product.

The present invention relates to a process for the preparation of the compound of formula **(I):** 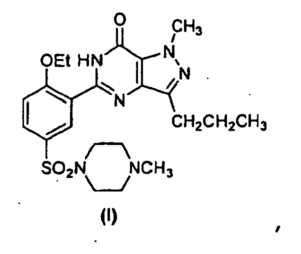 comprising the step of converting a compound selected from the group consisting of the compounds of formulae **(II), (III)** and **(IV):** 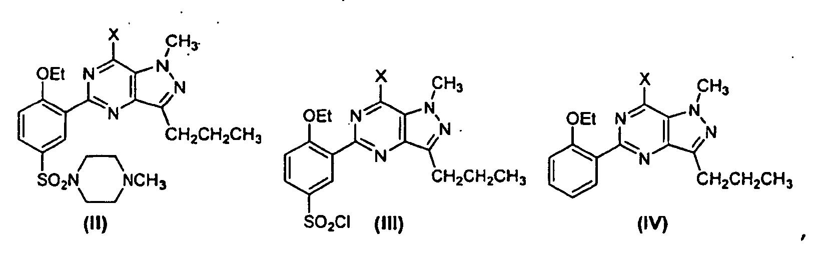 wherein X is halogen, preferably Cl or Br,
in one or more steps to give the compound of formula **(I).**

The present invention also relates to a process for the preparation of the compound of formula **(IV),** comprising
cyclizing the compound of formula **(V)** in the presence of POX₃, PX₃, PX₅ or their mixtures in any ratio: 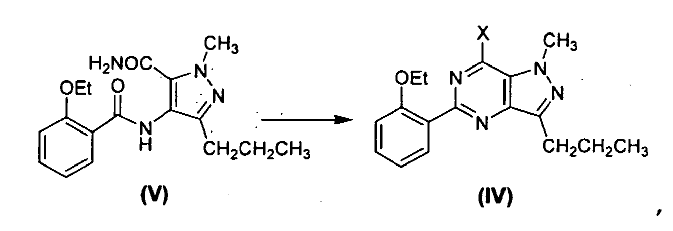 or
halogenating the compound of formula **(X)** in the presence of POX₃, PX₃, PX₅ or their mixtures in any ratio: 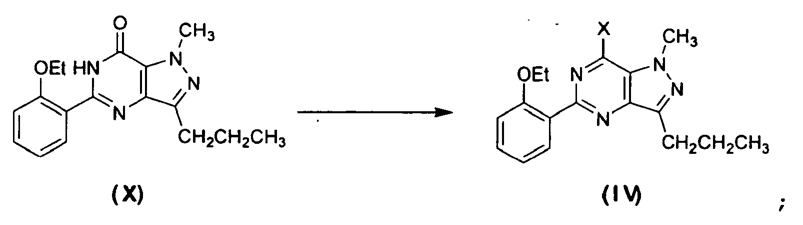 wherein X is halogen, preferably Cl or Br.

According to a preferred embodiment of the process for the preparation of the compound of formula **(IV),** the compound of formula **(IV)** is obtained
by treating the compound of formula **(V)** with POX₃, PX₃, PX₅ or their mixtures in any ratio at 50 - 120 °C, preferably 80 - 120 °C, optionally followed by pouring the reaction mixture into water, ice or their mixtures and collecting the precipitate which is the desired compound of formula **(IV),** or
by treating the compound of formula **(X)** with POX₃, PX₃, PX₅ or their mixtures in any ratio at 50 - 120 °C, preferably 80 - 120 °C, optionally followed by pouring the reaction mixture into water, ice or their mixtures and collecting the precipitate which is the desired compound of formula **(IV);**
wherein X is halogen, preferably Cl or Br.

The reaction can optionally be carried out in the presence of benzene, toluene, CH₂Cl₂, CHCl₃, ClCH₂CH₂Cl, THF, dioxane or their mixtures in any ratio.

The present invention also relates to a compound of formula **(III)** : 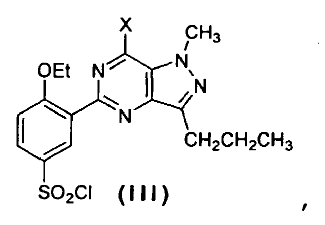 wherein X is halogen, preferably Cl or Br.

The present invention also relates to a process for the preparation of the compound of formula **(III),** comprising
chlorosulfonating the compound of formula **(IV)** in the presence of chlorosulfonic acid: 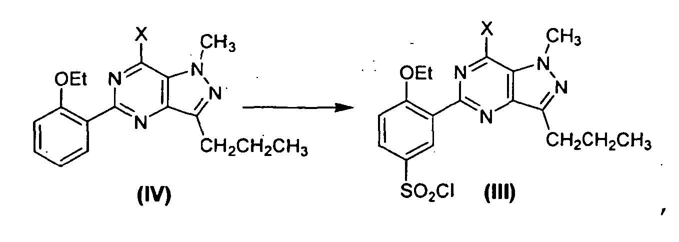 or
cyclizing the compound of formula **(VII)** in the presence of POX₃, PX₃, PX₅ or their mixtures in any ratio: 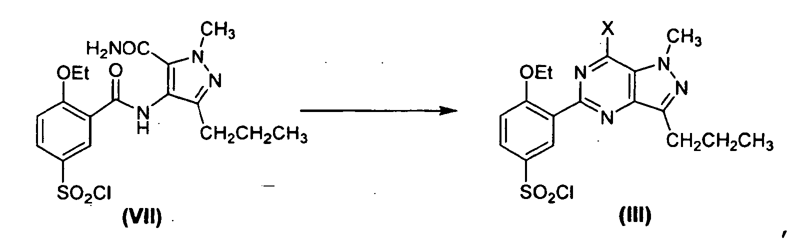 or
halogenating the compound of formula **(VIII)** in the presence of POX₃, PX₃, PX₅ or their mixtures in any ratio: 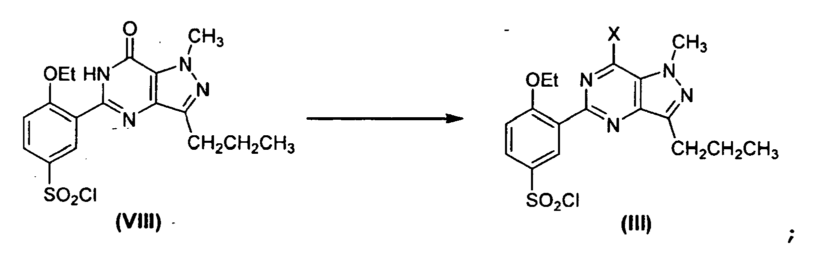 wherein X is halogen, preferably Cl or Br.

According to a preferred embodiment of the process for the preparation of the compound of formula **(III),** the compound of formula **(III)** is obtained
by treating the compound of formula **(IV)** with chlorosulfonic acid, preferably at a temperature of 0 - 50 °C, more preferably 0 - 25 °C, optionally followed by pouring the reaction mixture into water, ice or their mixtures and collecting the precipitate to give desired compound of formula **(III),** or
by heating the compound of formula **(VII)** in the presence of POX₃, PX₃, PX₅ or their mixtures in any ratio at 50 - 120 °C, preferably 80 - 120 °C, optionally followed by pouring the reaction mixture into water, ice or their mixtures and collecting the precipitate which is the desired compound of formula **(III),** or
by heating the compound of formula **(VIII)** in the presence of POX₃, PX₃, PX₅ or their mixtures in any ratio at 50 - 120°C, preferably 80 - 120 °C, optionally followed by pouring the re-action mixture into water, ice or their mixtures and collecting the precipitate which is the desired compound of formula **(III) ;**
wherein X is halogen, preferably Cl or Br.

In the process for the preparation of the compound of formula **(III),** the cyclization reaction and the halogenation reaction can optionally be carried out in the presence of benzene, toluene, CH₂Cl₂, CHCl₃, ClCH₂CH₂Cl, THF, dioxane or their mixtures in any ratio.

The present invention also relates to a process for the preparation of the compound of formula **(II),** comprising treating the compound of formula **(III)** with 1-methylpiperazine: 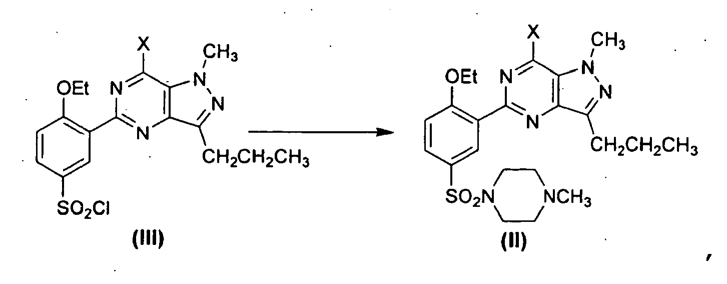 or
cyclizing the compound of formula **(VI)** in the presence of POX₃, PX₃, PX₅ or their mixtures in any ratio: 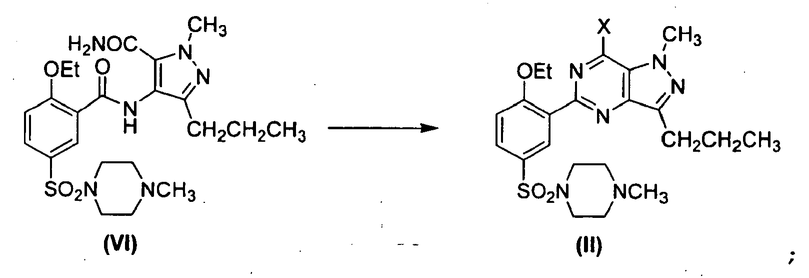 wherein X is halogen, preferably Cl or Br.

The compound of formula **(II)** can preferably be obtained
by dissolving the compound of formula **(III)** in a solvent selected from the group consisting of alkyl halides, lower aliphatic ketones and ethers, followed by adding a base and 1-methylpiperazine and collecting the desired compound from the reaction mixture, or
by cyclizing the compound of formula **(VI)** in the presence of POX₃, PX₃, PX₅ or their mixtures in any ratio.

The cyclization reaction mentioned above can optionally be carried out in the presence of benzene, toluene, CH₂Cl₂, CHCl₃, ClCH₂CH₂Cl, THF, dioxane or their mixtures in any ratio.

The present invention also relates to a process for the preparation of the compound of formula **(I),** wherein the compound of formula **(I)** can be obtained starting from the compound of formula **(II),** 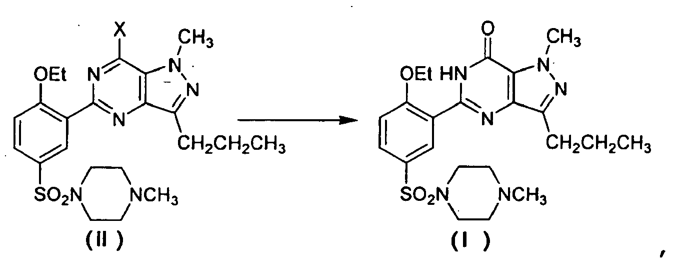 wherein X is halogen, preferably Cl or Br.

The above reaction can optionally be carried out in a solvent selected from the group consisting of water, methanol, ethanol, isopropanol, t-C₄H₉OH, glycol, ethylene glycol monomethyl ether or mixtures thereof. Furthermore, it can optionally be carried out by adding a base, such as an alkali metal alkoxide, an alkali metal or alkaline earth metal hydride, an amine, an amine metal derivative, a hydroxide, a carbonate, a bicarbonate or a mixture thereof in any ratio, or adding an acid such as hydrochloric acid, sulfuric acid, phosphoric acid, citric acid, tartaric acid, maleic acid or a mixture thereof.

In the process for the preparation of the compound of formula **(I),** the intermediate **(II)** can preferably be obtained by the above method.

According to a particular embodiment of the present invention, the process for the preparation of compound **(I)** is as outlined in scheme 1: 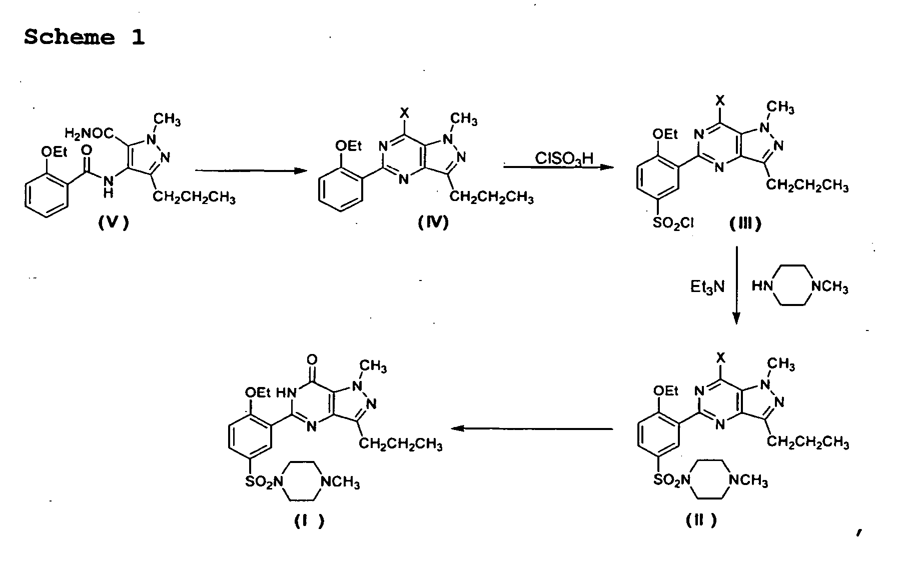 wherein X is halogen, preferably Cl or Br.

Compared to prior art processes, the present invention reduces side reactions, which are commonly found in alkaline catalyzed cyclizations and other steps in the preparation of sildenafil. The improvements provide for better yields and easier controlling of the reaction, which is thus particularly suitable for large scale preparation.

Starting from the compound of formula **(V),** the compound of formula **(III)** can be obtained via the intermediate **(IV)** or using a one-pot synthesis. According to the latter method, the compound of formula **(V)** is cylized followed by adding chlorosulfonation reagent without purifying the intermediate. The detailed and preferred procedure is as follows:

The compound of formula **(V)** is added into POX₃ or PX₃ in an ice bath. After 10 minutes, the mixture is heated slowly to 80 °C for 1 - 10 hours. When the reaction is complete, the mixture is cooled to room temperature and chlorosulfonic acid is added slowly. The mixture is stirred at room temperature for 1 - 5 hours and poured into water, crushed ice or a mixture thereof. The precipitated white solid is filtered and washed with ice water, followed by drying under vacuum to give the compound of formula **(III)** .

This reaction can optionally be carried out in an appropriate solvent which is selected from benzene, toluene, CH₂Cl₂, CHCl₃, ClCH₂CH₂Cl, THF, dioxane and mixtures thereof.

In the previously reported processes for the preparation of sildenafil, the cyclization is carried out in the presence of strong alkali, which usually results in side reactions, e.g. the isomerization of the pyrimidine skeleton and cleavage of the 5-ethoxy group on the benzene ring. Consequently, the yield is low. The present invention overcomes the disadvantages of traditional process. The cyclization is carried out in the presence of POX₃ or PX₃. The cylized product is stable. There is no side reaction of isomerization of the pyrimidine skeleton and cleavage of the ethoxy group on the benzene ring. Furthermore, in the subsequent step of chlorosulfonation, the remaining POX₃ can prevent hydrolysis of the compound of formula **(III).** Accordingly, the yield is greatly increased.

The compound of formula **(II)** is preferably obtained by treating the compound of formula **(III)** with 1-methylpiperazine in the presence of an acid removing agent. According to a particularly preferred embodiment, the compound of formula **(III)** is dissolved in an appropriate solvent and the acid removing agent is added. The reaction temperature is kept below 10 °C, and 1.1 equivalents of 1-methylpiperazine are slowly added. Stirring is continued for 1 - 3 hours at room temperature. Then, distilled water is added and the mixture is extracted with organic solvent. The organic phase is washed with saturated NH₄Cl and brine, and dried with Na₂SO₄. The solvent is evaporated in vacuo to give the desired compound of formula **(II)** as a white powder.

The appropriate solvent can be selected from alkyl halides, such as CH₂Cl₂, CHCl₃ or ClCH₂CH₂Cl; lower aliphatic ketones, such as acetone; ethers such as THF or ethylene glycol monomethyl ether; and mixtures thereof.

The acid removing agent can be selected from inorganic bases, such as carbonates, bicarbonates or hydroxides; or from organic bases, such as triethylamine.

The reaction for the preparation of sildenafil from the compound of formula **(II)** can be carried out under alkaline, neutral or acidic conditions. Preferably, the base is selected from alkali metal alkoxides, alkali metal hydrides, alkaline earth metal hydrides, amines, preferably triethylamine, amine metal derivatives, hydroxides, carbonates, bicarbonates- and mixtures thereof. Preferably, the acid is selected from hydrochloric acid, sulfuric acid, phosphoric acid, organic acids, such as citric acid, tartaric acid, maleic acid and mixtures thereof. Preferably, the solvent is selected from the group consisting of water and mixtures of water with methanol, ethanol, isopropanol, t-C₄H₉OH, glycol and/or ethylene glycol monomethyl ether. For example, the compound of formula **(II)** is dissolved in a mixed solvent of water and tert-butanol, to which one equivalent NaHCO₃ is added. The mixture is heated at 70 °C for 2 hours to give the compound of formula **(I).**

The compound of formula **(III)** can also be obtained by cyclization of the compound of formula **(VII)** or halogenation of the compound of formula **(VIII)** as follows: 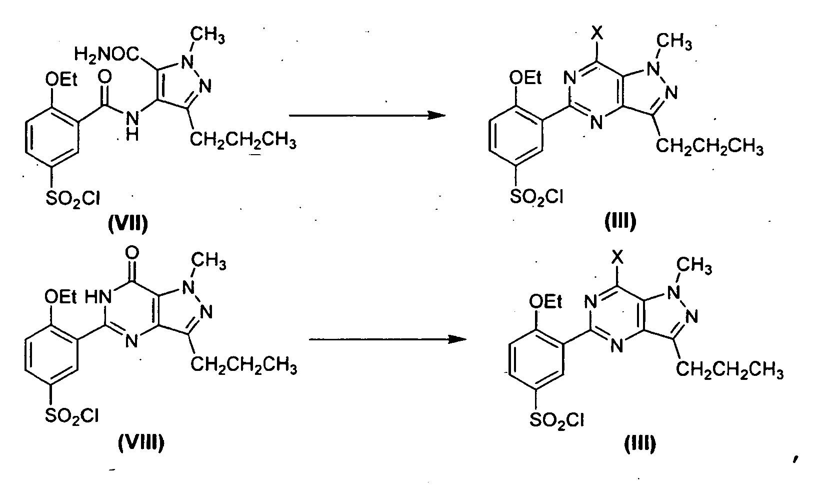 wherein X is halogen, preferably Cl or Br.

The compound of formula **(IV)** can also be obtained by halogenation of the compound of formula **(X):** 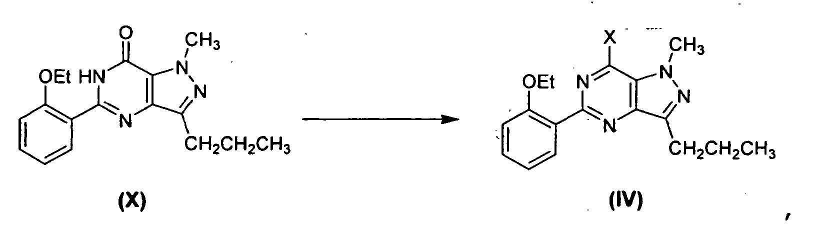 wherein X is halogen, preferably Cl or Br.

The three reactions mentioned immediately above can be carried out in the presence of POX₃, PX₃, PX₅ or mixtures thereof. Alternatively, they may be carried out in solvents such as benzene, toluene, CH₂Cl₂, CHCl₃, ClCH₂CH₂Cl or other solvents.

The compound of formula **(II)** can also be obtained from the compound of formula **(VI)** in the presence of POCl₃. 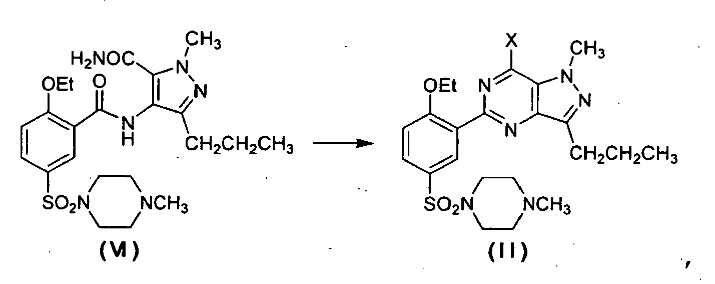 wherein X is Cl.

Regarding the preparation of the compounds of formulae **(VIII)** and **(X),** see also EP-A-0 463 756 (corresponding to CN1028758C). Regarding the preparation of the compound of formula **(VI),** see also EP-A-0 812 845 (corresponding to CN1106399C).

The intermediate **(V)** according to the present procedure can be prepared by conventional processes known in the literature. For example, it can be obtained by treating 2-ethoxybenzoyl chloride with 1-methyl-3-n-propyl-4-aminopyrazole-5-carboxamide **(XI):**

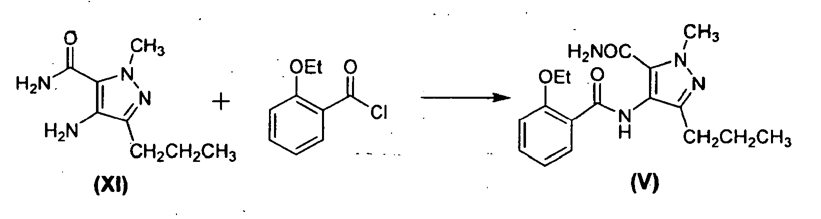

The compound of formula **(VII)** can be obtained from the compound of formula **(V)** by conventional processes known in the literature.

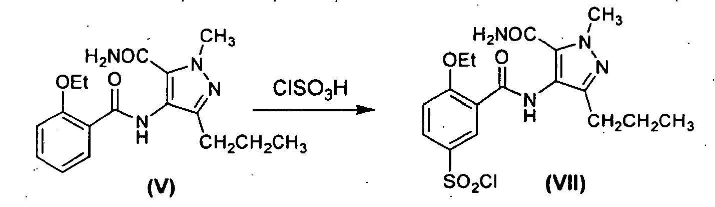

The compounds prepared according to the process of the present invention may exist in different crystal forms. A crystalline form of the compound of formula **(I),** i.e. sildenafil free base, is described in Melnikov et al., Journal of Pharmaceutical Sciences, 2003, 92, 2140-2143.

The process of the present invention may further comprise transforming the compound of formula **(I)** into a pharmaceutically acceptable salt or solvate thereof. A preferred salt of the compound of formula **(I)** is sildenafil citrate.

Processes for the preparation of pharmaceutically acceptable salts or solvates of the compound of formula **(I)** are generally known in the art. Such processes as well as different salts of sildenafil and polymorphic forms thereof are generally disclosed in Badwan et al., Analytical Profiles of Drug Substances and Excipients, Vol. 27, Academic Press, 2001; Admour et al., "Solid State Modification and Transformation in Sildenafil and Terfenadine Salts", MSc. Thesis, Jordan University of Science and Technology, Irbid, Jordan, January 1999; 46th APV Congress, Berlin, 3 to 6 April 2000, pages 639-640; WO-A-2004/072079; WO-A-2005/067936; WO-A-2007/080362; WO-A-2007/110559; and Journal of Shenyang Pharmaceutical University, 2002, 19, 173-175.

According to a preferred embodiment, transformation of the compound of formula **(I)** into sildenafil citrate is achieved by contacting the compound of formula **(I)** with citric acid in a suitable solvent, preferably methanol.

A particularly preferred process for the preparation of sildenafil citrate comprises the steps of:
(i) suspending sildenafil free base in methanol and heating the solution to 45 - 65 °C;
(ii) adding citric acid, preferably anhydrous citric acid, and methanol to the suspension of step (i), preferably at a temperature of 50 - 60 °C, and further heating the mixture to reflux to obtain a solution;
(iii) concentrating the solution to reduce the volume of the mixture, preferably to less than 50 %, more preferably to less than 35 % of its previous volume to obtain a suspension;
(iv) cooling the suspension to room temperature; and
(v) recovering the resulting crystals, preferably by filtration.

The invention also relates to a process for the preparation of a pharmaceutical composition comprising the compound of formula **(I),** or a pharmaceutically acceptable salt or solvate thereof, in combination with at least one pharmaceutically acceptable carrier, which includes the step of preparing the compound of formula **(I)** or the pharmaceutically acceptable salt or solvate thereof according to the processes according to the invention as described above.

Pharmaceutical preparations of sildenafil and sildenafil citrate can be prepared as generally known in the art. Suitable formulations are disclosed e.g. in EP-A-0 941 075, EP-A-0 960 621, JP-A-10298062, WO-A-2004/017976, WO-A-2004/072079 and IPCOM000146068D.

The following examples serve to further explain the invention without being intended to be limiting.

### Examples

Melting points were measured in open capillaries on a BUCHI-510 melting point apparatus and were uncorrected. EI-MS spectra were measured on a Finnigan MAT-95 spectrometer at 70 eV and an ion source temperature of 200; ¹H NMR spectra were determined in CDCl₃ solution on a Varian Mercury 300 spectrometer. All spectral results were in good agreement with the expected results. Conventional abbreviations are used for designation of major peaks: e.g. s, singlet; d, doublet; t, triplet; q, quartet; m, multiplet. As used herein, room temperature means 20 to 25 °C.

### Example 1

**Preparation 1:**

### 1-methyl-3-n-propyl-4-(2-ethoxybenzamido)-pyrazole-5-carboxamide (V)

In a 250 mL three-neck bottle, **(XI)** (20 g, 0.11 mol) was dissolved in dichloromethane (100 mL) and triethylamine (22.2 g, 0.22 mol) to prepare a solution in an ice bath. 2-ethoxybenzoyl chloride was added to the solution below 5 °C, and the mixture was stirred at room temperature for 2 hours. Water (40 mL) was added to stop the reaction, and the layers were separated. The organic phase was washed with brine (30 mL) and saturated aqueous sodium hydrogen carbonate solution, dried over anhydrous sodium sulfate, followed by concentration. The resulting residue was purified by re-crystallizing from ethyl acetate/petroleum ether to obtain **(V)** (31.5 g, yield 87 %) as a white solid. m.p. 153 - 154°C. ¹H NMR (CDCl₃, 300 MHz) δ: 0.93 (3H, t), 1.54 (3H, t), 1.65 (2H, m), 2.54 (2H, t), 4.06 (3H, s), 4.31 (2H, q), 5.62 (1H, br s), 7.05 (1H, d), 7.13 (1H, t), 7.54 (1H, t), 7.91 (1H, br s), 8.27 (1H, dd), 9.97 (1H, s).

### Preparation 1a:

### 1-methyl-3-n-propyl-4-(2-ethoxybenzamido)-pyrazole-5-carboxamide (V)

In a 250 mL three-neck bottle, **(XI)** (21.8 g, 0.10 mol) was dissolved in ethylacetate (200 mL) and triethylamine (32 ml, 0.23 mol) to prepare a solution in an ice bath. 2-ethoxybenzoyl chloride (17.0 mL, 0.11 mmol) was added to the solution below 5°C, and the mixture was stirred at room temperature for 2 hours. Water (170 mL) and petroleum ether (100 mL) were added to stop the reaction, and the mixture was stirred for another 0.5 h. The solid was filtrated and poured into water (170 mL) and stirred for 0.5 h, filtrated and dried (70 °C, 12 h) to afford compound (V) as a white solid (32.0 g, yield 95 %). m.p. 153 - 154 °C. The product was re-crystallized from ethyl acetate/petroleum ether. ¹H NMR (CDCl₃, 300 MHz) δ: 0.93 (3H, t), 1.54 (3H, t), 1.65 (2H, m), 2.54 (2H, t), 4.06 (3H, s), 4.31 (2H, q), 5.62 (1H, br s), 7.05 (1H, d), 7.13 (1H, t), 7.54 (1H, t), 7.91 (1H, br s), 8.27 (1H, dd), 9.47 (1H, s).

### Preparation 2:

### 4-[2-ethoxy-5-(chlorosulfonyl)benzamido]-1-methyl-3-n-propylpyrazole-5-carboxamide (VII)

The compound of formula **(V)** (5 g, 0.015 mol) was added portionwise to chlorosulfonic acid (10 mL) in an ice bath. The mixture was stirred for 30 minutes, and then the ice bath was removed. The reaction was continued for another 2 hours. The residue was poured into ice water. The resulting solid was collected by filtration, washed with ice water until the filtrate was neutral, and dried in vacuo to obtain **(VII)** (3.5 g) as a white solid in a yield of 54 %. ¹H NMR (CDCl₃, 300 MHz) δ: 0.94 (3H, t), 1.62 (3H, t), 1.66 (2H, m), 2.53 (2H, t), 4.06 (3H, s), 4.46 (2H, q), 5.72 (1H, s), 7.25 (1H, t), 7.62 (1H, s), 8.18 (1H, dd), 8.95 (1H, d), 9.20 (1H, s).

### Preparation 3:

### 7-chloro-1-methyl-5-(2-ethoxyphenyl)-3-n-propyl-1H-pyrazolo[4,3-d]pyrimidine (IV) (X=Cl)

### Method 1

(V) (5 g, 0.015 mol) was added into a 50 mL three-neck bottle in an ice bath. POCl₃ (20 mL) was added dropwise into the bottle to prepare a solution. The solution was heated at 80 °C for 2 hours, the mixture was poured into ice water to stop the reaction, and then the mixture was extracted with dichloromethane (3 x 30 mL). The organic phase was washed with brine (2 x 10 mL) and dried over anhydrous sodium sulfate (2 g), followed by concentration in vacuo to obtain **(IV)** (X=Cl, 4.1g, yield 82 %). ¹H NMR (CDCl₃, 300 MHz) δ: 1.03 (3H, t), 1.38 (3H, t), 1.90 (2H, m), 3.10 (2H, t), 4.15 (2H, q), 4.38 (3H, s), 7.03 (1H, d), 7.07 (1H, t), 7.42 (1H, t), 7.79 (1H, dd). EI-MS m/z 330 (M⁺, 76), 332 (25), 315 (52), 317 (15), 294(100), 296(32), 279(60), 261(28), 159(20).

### Method 2

**(X)** (5 g, 0.015 mol) was added into a 50 mL three-neck bottle in an ice bath. POCl₃ (20 mL) was added dropwise into the bottle to prepare a solution. The solution was heated at 80 °C for 2 hours, the mixture was poured into ice water to stop the reaction, and then the mixture was extracted with dichloromethane (3 x 30 mL). The organic phase was washed with brine (2 x 10 mL) and dried over anhydrous sodium sulfate (2 g), followed by concentration in vacuo to obtain **(IV)** (X=Cl, 4.4 g, yield 83 %) as a white solid.

### Method 3

**(V)** (5 g, 0.015 mol) was dissolved in benzene (20 mL). A solution of POCl₃ (2.8 mL) in benzene was added dropwise into the solution in an ice bath, and then the ice bath was removed. The reaction mixture was heated at 80 °C for 3 hours, and the benzene was removed by distillation under reduced pressure. The residue was poured onto ice-water (20 mL) and the solution was extracted with dichloromethane (3 x 30 mL). The organic phase was washed with brine (2 x 10 mL) and dried over anhydrous sodium sulfate (2 g), followed by concentration to obtain **(IV)** (X=Cl, 3.6 g, yield 72 %) as a white solid.

### Preparation 4:

### 7-chloro-1-methyl-5-[2-ethoxy-5-(chlorosulfonyl)phenyl]-3-n-propyl-1H-pyrazolo[4,3-d]pyrimidine (III) (X=Cl)

### Method 1

**(V)** (5 g, 0.015 mol) was added into a 50 mL three-neck bottle in an ice bath, and then POCl₃ (10 mL) was added. The reaction mixture was heated at 80 °C for 2 hours, and the thus obtained solution was cooled to a temperature below 5 °C. Chlorosulfonic acid (10 mL) was added into the reaction mixture. 30 minutes later, the ice bath was removed and the reaction mixture was further stirred at room temperature for 2 hours. The mixture was poured into ice water (20 mL). The resulting solid was collected by filtration, washed with ice water until the filtrate was neutral, and dried in vacuo for 3 hours to obtain **(III)** (X=Cl, 5.7 g) in a yield of 88 %, m.p. 155 - 156 °C. ¹H NMR (CDCl₃, 300 MHz) δ: 1.04 (3H, t), 1.43 (3H, t), 1.89 (2H, m), 3.09 (-2H, t), 4.27 (2H, q), 4.42 (3H, s), 7.19 (1H, d), 8.10 (1H, dd), 8.46 (1H, d). EI-MS m/z 430(31), 428 (M⁺, 41), 413(45), 415(34), 393(20), 357(24), 328(25), 292(100).

### Method 2

**(IV)** (X=Cl, 3 g) was added into a 50 mL flask, and then chlorosulfonic acid (6 mL) was added to prepare a solution in an ice bath. 30 minutes later, the ice bath was removed. The reaction mixture was further stirred at room temperature for 2 hours, and then the mixture was poured into ice water (15 mL). The resulting solid was collected by filtration, washed with ice water until the filtrate was neutral, and dried under vacuum at 35 °C for 3 hours to obtain **(III)** (X=Cl, 2.8 g, yield 72 %).

### Method 3

**(VII)** (5 g, 0.012 mol) was added into a 50 mL flask, and POCl₃ (10 mL) was added slowly to prepare a solution. The reaction mixture was heated at 80 °C for 2 hours and then poured into ice water (20 mL), the resulting solid was collected by filtration and washed with ice water until the filtrate was neutral, and dried in vacuo to obtain **(III)** (X=Cl, 3.2 g, yield 64 %).

### Method 4

**(VIII)** (5 g, 0.012 mol) was added into a 50 mL flask, POCl₃ (10 mL) was added slowly to prepare a solution, the reaction mixture was heated at 80 °C for 2 hours and then poured into ice-water (20 mL). The resulting solid was collected by filtration, washed with ice water until the filtrate was neutral, and dried in vacuo to obtain **(III)** (X=Cl, 4.8 g, yield 92 %) as a white solid.

### Method 5

**(VIII)** (5 g, 0.012 mol) was added into a 50 mL flask, POCl₃ (10 mL) was added slowly to prepare a solution, the reaction mixture was heated at 70 °C for 2 hours and then poured into ice water (20 mL). The resulting solid was collected by filtration, washed with ice water until the filtrate was neutral, and dried in vacuo to obtain **(III)** (X=Cl, 4.8 g, yield 92 %) as a white solid.

### Method 6

**(VIII)** (5 g, 0.012 mol) was dissolved in benzene (40 mL). PCl₅ was added into the solution in an ice bath. 30 minutes later, the ice bath was removed, the reaction mixture was heated at 90 °C for 2 hours and then poured into ice water (20 mL). The resulting solid was collected by filtration, washed with ice water until the filtrate was neutral, and dried in vacuo to obtain **(III)** (X=Cl, 3.5 g, yield 67 %) as a white solid.

### Preparation 5:

### 7-chloro-1-methyl-5-[2-ethoxy-5-(4-methylpiperazinylsulfonyl)-phenyl]-3-n-propyl-1H-pyrazolo[4,3-d]pyrimidine (II) (X=Cl)

### Method 1

**(III)** (X=Cl, 2 g) was dissolved in dichloromethane (20 mL) and triethylamine (0.94 g, 9.3 mmol) to prepare a solution. A solution of 1-methylpiperazine (0.51 g, 5.1 mmol) in dichloromethane (5 mL) was then added dropwise to the solution in an ice bath. The ice bath was removed, and the mixture was stirred at room temperature for 2 hours. Water was added, and the layers were separated. The organic phase was washed with saturated aqueous ammonium chloride solution (2 x 5 ml) and brine (2 x 5 mL) and dried over anhydrous Na₂SO₄ (1 g) for 30 minutes. The solvent was removed by distillation under reduced pressure to afford crude **(II)** as a white solid. The crude product was purified by re-crystallizing from dichloromethane/petroleum ether to obtain **(II)** (X=Cl, 1.95 g, yield 85 %) as white needle crystals, m.p. 157 - 159 °C. ¹H NMR (CDCl₃, 300 MHz) δ: 1.03 (3H, t), 1.41 (3H, t), 1.90 (2H, m), 2.41 (3H, s), 2.69 (4H, s), 3.04 (2H, t), 3.23 (4H, s), 4.19 (2H, q), 4.37 (3H, s), 7.10 (1H, d), 7.78 (1H, dd), 8.17 (1H, s) EI-MS m/z 494 (2), 492 (M⁺, 6), 424 (2), 422 (7), 330 (7), 99(100).

### Method 2

**(VI)** (2 g, 0.004 mol) was added into a 50 mL flask. POCl₃ (10 mL) was added slowly to prepare a solution. The reaction mixture was heated at 80 °C for 2 hours and then poured into ice water (8 mL). The resulting white solid was collected by filtration, washed with ice water until the filtrate was neutral, and dried in vacuo to obtain **(II)** (X=Cl, 1.5 g, yield 75%) as a white solid.

### Method 3

**(VI)** (2 g, 4 mmol) was dissolved in benzene (10 mL). A solution of POCl₃ (0.65 mL) in benzene (2 mL) was added slowly to the solution, the reaction mixture was heated at 80 °C for 2 hours and then poured into ice water (8 mL). The resulting white solid was collected by filtration, washed with ice water until the filtrate was neutral, and dried under vacuum to obtain **(II)** (X=Cl, 1.4 g, yield 70 %) as a white solid.

### Preparation 6:

### 1-methyl-5-[2-ethoxy-5-(4-methylpiperazinylsulfonyl)phenyl]-3-n-propyl-1,6-dihydro-7H-pyrazolo[4,3-d]pyrimidin-7-one (I)

### Method 1

**(II)** (X=Cl, 1 g) was dissolved in tert-butyl alcohol (3 mL) and water (3 mL) to prepare a solution. NaHCO₃ (0.17 g, 2.1 mmol) was added into the solution, and the mixture was heated to reflux for 2 hours. The tert-butyl alcohol was removed by distillation under reduced pressure. The pH value of solution was adjusted to 8.5 - 9.5 by adding 1 mol/L HCl aqueous solution in an ice water bath. The resulting white solid was collected by filtration and purified by re-crystallizing from ethanol to obtain **(I)** (0.87 g, yield 90 %) as white needle crystals. m.p. 186 - 188 °C; ¹H. NMR (CDCl₃, 300 MHz), δ: 1.01 (3H, t), 1.63 (3H, t), 1.85 (2H, m), 2.27 (3H, s), 2.50 (4H, t), 2.92 (2H, t), 3.10 (4H, t), 4.27 (3H, s), 4.37 (2H, q), 7.14 (1H, d), 7.83 (1H, dd), 8.83 (1H, d), 10.81 (1H, s) EI-MS m/z 474 (M⁺, 4), 410 (8), 404 (58), 312 (7), 99(100) .

### Method 2

**(II)** (X=Cl, 1 g) was dissolved in water (5 mL), and the mixture was heated to reflux for 4 hours. Dichloromethane (15 mL) was added to the solution, and the layers were separated. The organic phase was washed with brine (2 x 5 mL) and dried over anhydrous Na₂SO₄ for 30 minutes. The dichloromethane was removed by distillation under reduced pressure to **afford (I)** (0.80 g, yield 83 %) as a white solid.

### Method 3

**(II)** (X=Cl, 1 g) was dissolved in a solution of 1 mol/L aqueous HCl aqueous solution (5 mL), and the reaction mixture was heated at 60 °C for 2 hours. The pH value of solution was adjusted to 8.5 - 9.5 by adding NaHCO₃ in an ice water bath. The resulting white solid was collected by filtration and purified by re-crystallizing from ethanol to obtain **(I)** (0.76 g, yield 79 %) as a white powder.

### Preparation 7:

### 7-bromo-1-methyl-5-(2-ethoxyphenyl)-3-n-propyl-1H-pyrazolo[4,3-d]pyrimidine (IV) (X=Br)

The title compound was prepared by PBr₃ replacing POCl₃ following the procedure of Method 2 in Preparation 3 to afford **(IV)** (X=Br, 4.0 g) in a yield of 67 %.

### Preparation 8:

### 7-bromo-1-methyl-5-[2-ethoxy-5-(chlorosulfonyl)phenyl]-3-n-propyl-1H-pyrazolo[4,3-d]pyrimidine (III) (X=Br).

### Method 1

The title compound was prepared by PBr₃ replacing POCl₃ following the procedure of Method 1 in Preparation 4 to obtain **(III)** (X=Br, 5.0 g) in a 70% yield. ¹H NMR (CDCl₃, 300 MHz) δ: 1.03 (3H, t), 1.43 (3H, t), 1.88 (2H, m), 3.04 (2H, t), 4.23 (2H, q), 4.40 (3H, s), 7.15 (1H, d), 8.07 (1H, dd), 8.45 (1H, d) EI-MS m/z 475(8), 473(M⁺+1, 8), 382(24), 380 (24), 292(76), 82(98), 80(100), 79(40).

### Method 2

The title compound was prepared by POBr₃ replacing POCl₃ following the procedure of Method 3 in Preparation 4 to obtain **(III)** (X=Br, 3.4 g) in a yield of 59 %.

### Method 3

The title compound was prepared by PBr₅ replacing PCl₅ following the procedure of Method 6 in Preparation 4 to obtain **(III)** (X=Br, 3.0 g) in a yield of 52 %.

### Preparation 9:

### 7-bromo-1-methyl-5-[2-ethoxy-5-(4-methylpiperazinylsulfonyl)-phenyl]-3-n-propyl-1H-pyrazolo[4,3-d]pyrimidine (II) (X=Br)

The title compound was prepared from 7-bromo-1-methyl-5-[2-ethoxy-5-(chlorosulfonyl)phenyl]-3-n-propyl-1*H-*pyrazolo[4,3-d]pyrimidine **((III),** X=Br) following the procedure of Method 1 in Preparation 5 to obtain **(II)** (X=Br, 0.8 g) in a yield of 72 %. ¹H NMR (CDCl₃, 300 MHz) δ: 1.03 (3H, t), 1.43 (3H, t), 1.87 (2H, m), 2.51 (3H, s), 2.83(4H, s), 3.04 (2H, t), 3.50 (4H, s), 4.21 (2H, q), 4.39 (3H, s), 7.14 (1H, d), 7.77 (1H, dd), 8.20 (1H, s) EI-MS m/z 538 (M⁺, 6), 536(6), 468(6), 466(6), 456(20), 99(100).

### Preparation 10:

### 1-methyl-5-[2-ethoxy-5-(4-methylpiperazinylsulfonyl)phenyl]-3-n-propyl-1,6-dihydro-7H-pyrazolo[4,3-d]pyrimidin-7-one (I)

The title compound was prepared from 7-bromo-1-methyl-5-[2-ethoxy-5-(4-methylpiperazinylsulfonyl)phenyl]-3-n-propyl-1,6-dihydro-7*H-*pyrazolo[4,3-d]pyrimidine following the procedure of Method 1 in Preparation 6 to obtain **(I)** (0.85 g) in a yield of 75 %.

### Example 2:

### Preparation of the compound of formula (I):

**Step 1. (V)** (5 g, 0.015 mol) was added into a 50 mL flask, and POCl₃ (10 mL) was added slowly to prepare a solution. The mixture was heated at 120 °C for 1 hour, the reaction mixture was poured into ice water (30 mL) to stop the reaction, and then the mixture was extracted with dichloromethane (3 x 30-mL). The organic phase was washed with brine (2 x 10 mL) and dried over anhydrous sodium sulfate for 30 minutes, followed by concentration to obtain **(IV)** (X=Cl, 4.1 g) as a white solid.
**Step 2. (IV)** (X=Cl, 5 g, 0.015 mol) was added portionwise to chlorosulfonic acid (10 mL) in an ice bath. The mixture was stirred in the ice bath for 30 minutes, then the ice bath was removed. The reaction was continued for another 2 hours. The residue was poured into water. The resulting solid was collected by filtration, washed with ice water until the filtrate was neutral, and dried in vacuo to obtain **(III)** (X=Cl) as a white solid.
**Step 3. (III)** (X=Cl, 2 g) was dissolved in dichloromethane (20 mL) and triethylamine (0.94 g, 9.3 mmol) to prepare a solution. A solution of 1-methylpiperazine (0.51 g, 5.1 mmol) in dichloromethane (5 mL) was then added dropwise to the solution in an ice bath. The ice bath was removed, and the mixture was stirred at room temperature for 2 hours. Water was added and the layers were separated. The organic phase was washed with saturated aqueous ammonium chloride solution (2 x 10 ml) and brine (2 x 10 mL) and dried over anhydrous Na₂SO₄ (1 g) for 30 minutes. The solvent was removed by distillation under reduced pressure to afford crude **(II)** (X=Cl) as a white solid. The crude product was purified by re-crystallizing from dichloromethane/petroleum ether to obtain **(II)** (X=Cl) as a white crystalline solid.
**Step 4. (II)** (X=Cl, 1 g) was dissolved in tert-butyl alcohol (3 mL) and an appropriate amount of water to prepare a solution. NaHCO₃ (2.1 mmol) was added into the solution, and the mixture was heated at 90 °C for 1.5 hours. The tert-butyl alcohol was removed by distillation under reduced pressure at 40 °C. The pH value of the solution was adjusted to 8.5 - 9.5, and the resulting white solid was collected by filtration and purified by re-crystallizing with ethanol to obtain **(I)** (0.87 g) as a white needle crystal.

**Alternative A:** Step 1 could be followed immediately by step 2 without purifying the intermediate, the detailed procedure being as follows:

**(V)** (5 g, 0.015 mol) was added into a 50 mL three-neck bottle. POCl₃ (10 mL) was added to prepare a solution in an ice bath, and the reaction mixture was heated at 120 °C for 1 hour, and then the resulting solution was cooled to a temperature below 0 °C. Chlorosulfonic acid (10 mL) was added into the reaction mixture. 30 minutes later, the ice bath was removed, and the reaction mixture was stirred at the room temperature for 2 hours. The mixture was poured into ice water (30 mL), the resulting white solid was collected by filtration, washed with ice water until the filtrate was neutral, and dried in vacuo for 3 hours at 35 °C to obtain **(III)** (X=Cl, 5.7 g).

### Example 3:

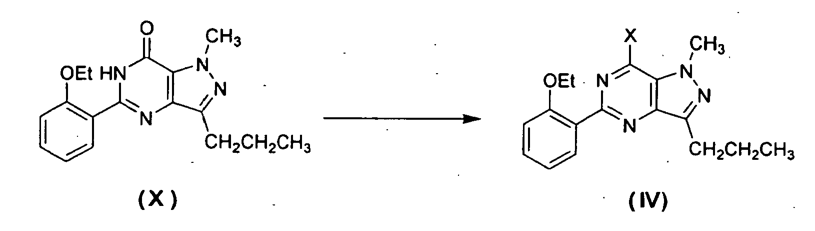

**(X)** (5 g, 0.016 mol) was added into a 50 mL three-neck bottle in an ice bath. POCl₃ (10 mL) was added dropwise into the bottle to prepare a solution. The solution was heated for 4 hours at 50 °C, the mixture was poured onto ice to stop the reaction, and then the mixture was extracted with dichloromethane (3 x 30 mL). The organic phase was washed with brine (2 x 10 mL) and dried over anhydrous sodium sulfate (2 g) for 30 minutes, hollowed by concentration to obtain **(IV)** (X=Cl, 4.4 g) as a white solid.

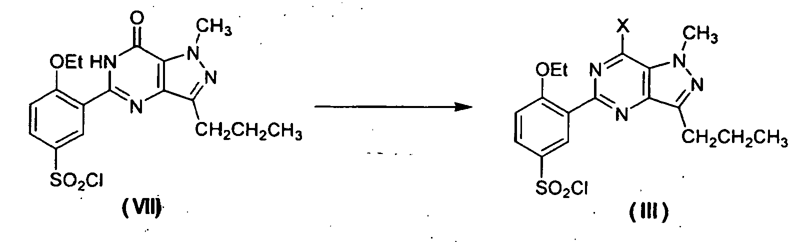

**(VIII)** (5 g, 0.012 mol) was added into a 50 mL flask, and POCl₃ (10 mL) was added slowly to prepare a solution. The reaction mixture was heated at 60 °C for 3 hours and then poured into water (20 mL). The resulting solid was collected by filtration, washed with ice water until the filtrate was neutral, and dried in vacuo for 2 hours at 35 °C to obtain **(III)** (X=Cl, 4.8 g) as a white solid.

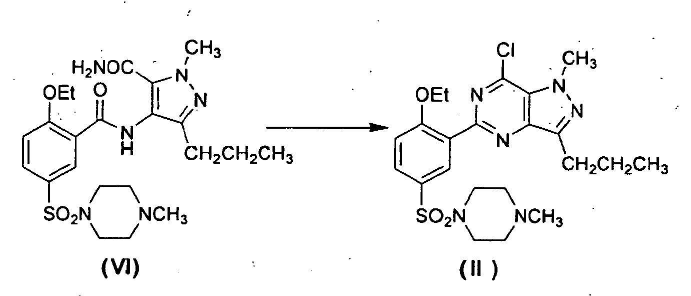

**(VI)** (2 g, 0.004 mol) was added into a 50 mL flask. POCl₃ (10 mL) was added slowly to prepare a solution. The reaction mixture was heated at 100 °C for 1.5 hours and then poured onto crushed ice. The resulting white solid was collected by filtration, washed with ice water until the filtrate was neutral, and dried in vacuo for 2 hours at 35 °C to obtain **(II)** (X=Cl, 1.5 g) as a white solid.

### Example 4:

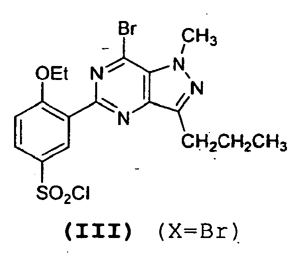

The compound of formula **(III)** (X=Br) was prepared by PBr₃ replacing POCl₃ following the procedure of Alternative A in Example 2 to obtain **(III)** (X=Br, 5.0 g).

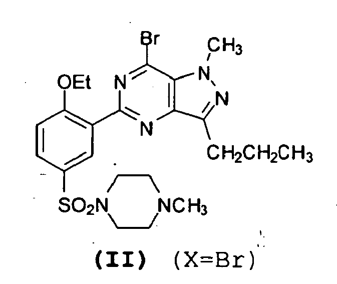

The compound of formula **(II)** (X=Br) was prepared from **(III)** (X=Br, 1.0 g) following the procedure of Step 3 in Example 2 to obtain **(II)** (X=Br, 0.8 g).

The compound of formula **(I)** was prepared from **(II)** (X=Br, 0.5 g) according to the procedure of Step 4 in Example 2 to obtain **(I)** (0.36 g) as white needle crystals.

### Example 5

### Preparation of sildenafil citrate

### Example 5.1:

A suspension of sildenafil (4.75 g, 10 mmol) in methanol (20 mL) was heated to 45 - 65 °C. Citric acid monohydrate (2.10 g, 10 mmol) was added to the suspension and a solution was obtained. After a short period of time, precipitation occurred. The precipitate was cooled, collected by filtration and washed with methanol to obtain 6.6 g of sildenafil citrate. The precipitate was re-crystallized from methanol (160 mL). White needle crystals of sildenafil citrate (4.5 g) were obtained.

### Example 5.2:

A suspension of sildenafil (4.75 g, 10 mmol) in methanol (20 mL) was heated to 45 - 65 °C. Citric acid monohydrate (2.10 g, 10 mmol) was added to the suspension and a solution was obtained. After a short period of time precipitation occurred. The precipitate was then cooled and methanol (150 mL) was added. The resulting suspension was heated to reflux to obtain a solution. The solution was cooled and crystallization occurred. Crystals were filtered and washed with methanol. White needle crystals of sildenafil citrate (5.2 g) were obtained.

### Example 5.3:

A suspension of sildenafil (4.75 g, 10 mmol) in methanol (20 mL) was heated to 45 - 65 °C. Citric acid anhydrous (1.90 g, 10 mmol) was added to the suspension to obtain a solution. After a short period of time precipitation occurred. The precipitate was then cooled, collected by filtration and washed with methanol to obtain 6.4 g of sildenafil citrate. The precipitate was re-crystallized from methanol (160 mL). White needle crystals of sildenafil citrate (4.6 g) were obtained.

### Example 5.4:

A suspension of sildenafil (4.75 g, 10 mmol) in methanol (20 mL) was heated to 45 - 65 °C. Citric acid anhydrous (1.90 g, 10 mmol) was added to the suspension and a solution was obtained. After a short period of time precipitation occurred. The precipitate was then cooled and methanol (150 mL) was added. The resulting suspension was heated to reflux to obtain a solution. The solution was cooled and crystallization occurred. The crystals were filtered and washed with methanol. White needle crystals of sildenafil citrate (5.2 g) were obtained.

### Example 5.5:

A suspension of sildenafil (4.75 g, 10 mmol) and citric acid (2.1 g) in methanol (20 mL) was heated to reflux for 1 hour and then cooled to 20 - 25 °C. Precipitation occurred and the precipitate was collected by filtration and washed with methanol. The precipitate was re-crystallized from methanol. White needle crystals of sildenafil citrate (5.7 g) were obtained.

### Example 5.6:

### Process for yield improvement for crystallization with concentration of solution or suspension

A suspension of sildenafil (4.75 g, 10 mmol) in methanol (20 mL) was heated to 45 - 65 °C. Citric acid anhydrous (1.90 g, 10 mmol) and methanol (150 mL) were added to the suspension at 50 -60 °C, and a solution was obtained at reflux. The solution was then concentrated to a volume of 50 - 60 mL. Precipitation occurred during the concentration. The suspension was cooled to room temperature and crystals were filtered and washed with methanol. The product was dried in a vacuum dryer at 60 °C. White needle crystals of sildenafil citrate (6.3 g) were obtained.

### Example 5.7:

A suspension of sildenafil (4.75 g, 10 mmol) in methanol (20 mL) was heated to 45 - 65 °C. Citric acid anhydrous (1.90 g, 10 mmol) was added to the suspension, and a solution was obtained. After a short period of time precipitation occurred. Methanol (150 mL) was added and the suspension was heated to reflux to obtain a solution. The solution was then concentrated and cooled to 20 - 25 °C to obtain white needle crystals of sildenafil citrate (5.8 g).

### Example 5.8:

### Crystallization of small particle size white needle crystals of sildenafil citrate.

A suspension of sildenafil (4.75 g, 10 mmol) in methanol (20 mL) was heated to 45 - 65 °C. Citric acid anhydrous (1.90 g, 10 mmol) and methanol (150 mL) were added to the suspension and a solution was obtained. The solution was quickly cooled to 20 - 25 °C and crystallization occurred. Crystals were filtered and washed with methanol. Small size white needle crystals of sildenafil citrate (5.2 g) were obtained.

### Example 5.9:

### Crystallization of small particle size white needle crystals of sildenafil citrate.

A suspension of sildenafil (4.75 g, 10 mmol) in methanol (20 mL) is heated to 45 - 65 °C. Citric acid anhydrous (1.90 g, 10 mmol) and methanol (150 mL) were added to the suspension and a solution was obtained. The solution was concentrated and quickly cooled to 20 -25 °C and crystallization occurrred. Crystals were filtered and washed with methanol. Small size white needle crystals of sildenafil citrate (5.8 g) were obtained.

### Example 5.10:

### Crystallization of large particle size white needle crystals of sildenafil citrate

A suspension of sildenafil (4.75 g, 10 mmol) in methanol (20 mL) was heated to 45 - 65 °C. Citric acid anhydrous (1.90 g, 10 mmol) and methanol (150 mL) were added to the suspension and a solution was obtained. The solution was concentrated and cooled to 20 - 25 °C and crystallization occurred. The suspension was alternately heated to 55 °C and cooled to 20 °C, several times. The obtained crystals were filtered and washed with methanol. Large size white needle crystals of sildenafil citrate (5.2 g) were obtained.

### Example 5.11:

### Preparation of sildenafil citrate

A suspension of sildenafil (4.75 g, 10 mmol) in methanol (20 mL) was heated to 45 - 65 °C under nitrogen atmosphere. Citric acid (1.90 g, 10 mmol) and methanol (150 mL) were added to the suspension and a solution was obtained. The solution was cooled to -20 - 25 °C and crystallization occurred. The suspension was then filtered under nitrogen to obtain white needle crystals of sildenafil citrate (5.2 g).

### Example 6:

### Determination of purity of sildenafil

High resolution HPLC was used to determine sildenafil and sildenafil citrate related impurities. The tests were carried out on Hypersil MOS C₈, 200 mm x 4.6 mm i.d. (particle size 5 µm). The mobile phase was A: 0.1% triethylamine in 0.025 M KH₂PO₄ (pH 6.0, adjusted with orthophosphoric acid) and B: acetonitrile. Gradient elution was used: 30 % B (0 min), 30 % B (3 min), 70 % B (20 min), 70 % B (30 min). The chromatograph was equipped with a UV detector.

Sildenafil prepared according to example 2, step 4 was determined to have a purity of 99.95 %.

## Claims

1. A process for the preparation of the compound of formula **(I) :** 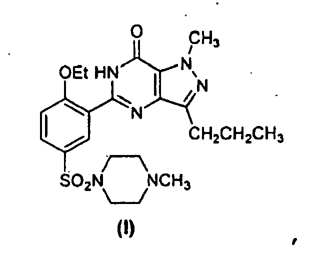 comprising the step of converting a compound selected from the group consisting of the compounds of formulae **(II), (III)** and **(IV):** 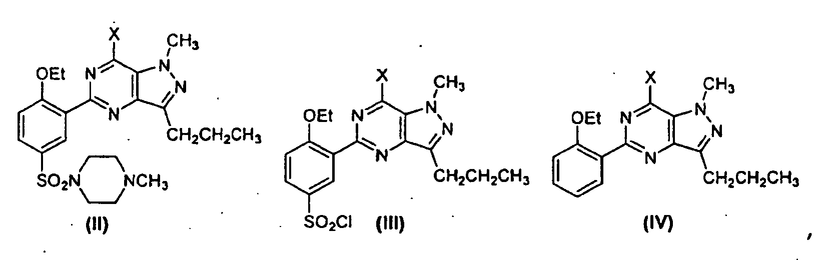 wherein X is halogen,
in one or more steps to give the compound of formula **(I).**

2. The process according to claim 1, wherein X is Cl or Br.

3. The process according to claim 1 or 2, wherein the compound of formula **(IV)** is obtained
by cyclizing the compound of formula **(V)** in the presence of POX₃, PX₃, PX₅ or mixtures thereof: 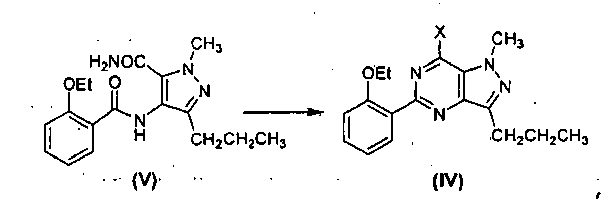 or
by halogenating the compound of formula **(X)** in the presence of POX₃, PX₃, PX₅ or mixtures thereof:

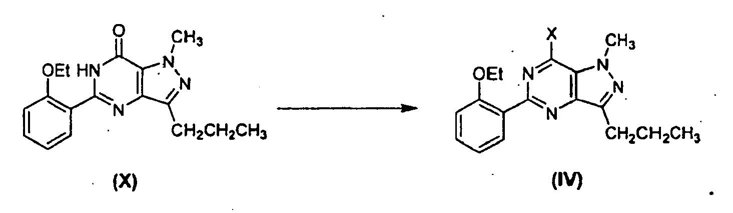

4. The process according to claim 3, wherein the compound of formula **(IV)** is obtained
by treating the compound of formula **(V)** with POX₃, PX₃, PX₅ or mixtures thereof at 50 - 120 °C, or
by treating the compound of formula **(X)** with POX₃, PX₃, PX₅ or mixtures thereof at 50 - 120°C.

5. The process according to claim 4, wherein the treatment is followed by pouring the reaction mixture into water, ice or mixtures thereof and collecting the precipitate to obtain the compound of formula **(IV).**

6. The process according to any one of claims 3 to 5, wherein the cyclisation reaction or the halogenation reaction is carried out in the presence of benzene, toluene, CH₂Cl₂, CHCl₃, ClCH₂CH₂Cl, THF, dioxane or mixtures thereof.

7. The process according to any one of claims 1 to 6, wherein the compound of formula **(III)** is obtained by chlorosulfonating the compound of formula **(IV)** in the presence of chlorosulfonic acid:

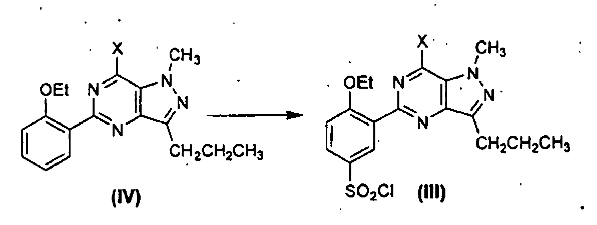

8. The process according to any one of claims 1 to 6, wherein the compound of formula **(III)** is obtained by cyclizing the compound of formula **(V)** in the presence of POX₃, PX₃, PX₅ or mixtures thereof, followed by chlorosulfonating in the presence of chlorosulfonic acid without purifying the intermediate.

9. The process according to claim 1 or 2, wherein the compound of formula **(III)** is obtained
by cyclizing the compound of formula **(VII)** in the presence of POX₃, PX₃, PX₅ or mixtures thereof: 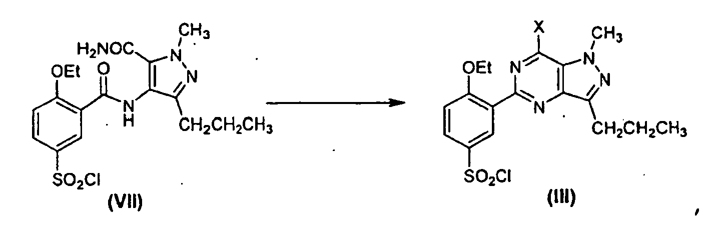 or
by halogenating the compound of formula **(VIII)** in the presence of POX₃, PX₃, PX₅ or mixtures thereof:

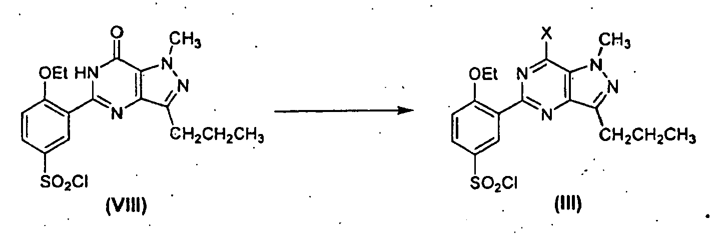

10. The process according to claim 7, wherein the compound of formula **(III)** is obtained by treating the compound of formula **(IV)** with chlorosulfonic acid at 0 - 50 °C.

11. The process according to claim 9, wherein the compound of formula **(III)** is obtained
by heating the compound of formula **(VII)** in the presence of POX₃, PX₃, PX₅ or mixtures thereof at 50 - 120 °C, or
by heating the compound of formula **(VIII)** in the presence of POX₃, PX₃, PX₅ or mixtures thereof at 50 - 120 °C.

12. The process according to claim 9 or 11, wherein the cyclization reaction or the halogenation reaction is carried out in the presence or benzene, toluene, CH₂Cl₂, CHCl₃, ClCH₂CH₂Cl, THF, dioxane or mixtures thereof.

13. The process according to any one of claims 1 to 12, wherein the compound of formula **(II)** is obtained by treating the compound of formula **(III)** with 1-methylpiperazine;

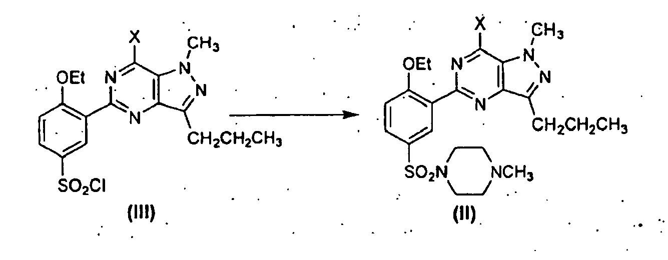

14. The process according to claim 1 or 2, wherein the compound of formula **(II)** is obtained by cyclizing the compound of formula **(VI)** in the presence of POX₃, PX₃, PX₅ or mixtures thereof:

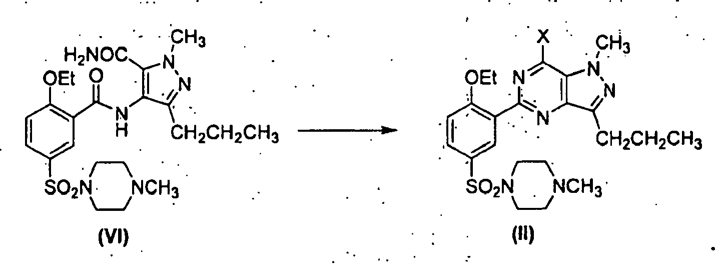

15. The process according to claim 13, wherein the compound of formula **(II)** is obtained by treating the compound of formula **(III)** with 1-methylpiperazine in the presence of an acid removing agent.

16. The process according to claim 14, wherein the compound of formula **(II)** is obtained by heating the compound of formula **(VI)** in the presence of POX₃, PX₃, PX₅ or their mixtures in any ratio at 50 - 120 °C.

17. The process according to claim 16, wherein the treatment is followed by pouring the reaction mixture into water, ice or mixtures thereof and collecting the precipitate.

18. The process according to claim 16 or 17, wherein the cyclization is carried out in the presence of benzene, toluene, CH₂Cl₂, CHCl₃, ClCH₂CH₂Cl, THF, dioxane or mixtures thereof.

19. The process according to any one of claims 1 to 18, wherein the compound of formula **(I)** is obtained by reacting the compound of formula **(II)** to give the compound of formula **(I)**:

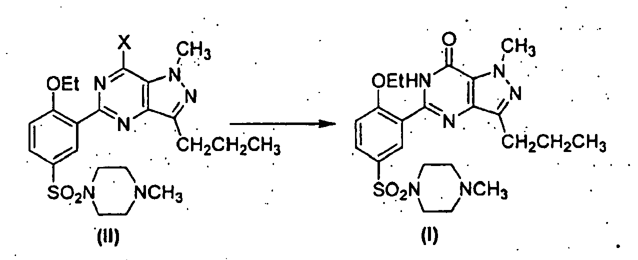

20. The process according to claim 19, wherein the reaction is carried out in a solvent selected from water, methanol, ethanol, isopropanol, t-C₄H₉OH, glycol, ethylene glycol monomethyl ether and mixtures thereof.

21. The process according to claim 19 or 20, wherein the reaction is carried out in the presence of a base or an acid.

22. The process according to claim 21, wherein the base is selected from alkali metal alkoxides, alkali metal hydrides, alkaline earth metal hydrides, amines, amine metal derivatives, hydroxides, carbonates, bicarbonates and mixtures thereof.

23. The process according to claim 21, wherein the acid is selected from hydrochloric acid, sulfuric acid, phosphoric acid, citric acid, tartaric acid, maleic acid or mixtures thereof.

24. The process according to any one of claims 1 to 23, further comprising transforming the compound of formula **(I)** into a pharmaceutically acceptable salt or solvate thereof.

25. The process according to claim 24, wherein the pharmaceutically acceptable salt is sildenafil citrate.

26. The process according to claim 25, wherein transforming of the compound of formula **(I)** into sildenafil citrate is achieved by contacting the compound of formula **(I)** with citric acid in a suitable solvent, preferably methanol.

27. A process for the preparation of a pharmaceutical composition comprising the compound of formula **(I),** or a pharmaceutically acceptable salt or solvate thereof, in combination with at least one pharmaceutically acceptable carrier, which includes the step of preparing the compound of formula **(I)** or the pharmaceutically acceptable salt or solvate thereof according to the process of any one of claims 1 to 26.

28. A compound of formula (III): 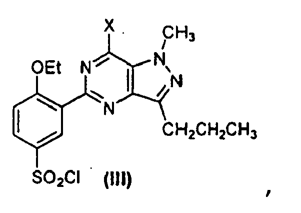 wherein X is Cl or Br.

## Patentansprüche

1. Verfahren zur Herstellung der Verbindung der Formel **(I):** 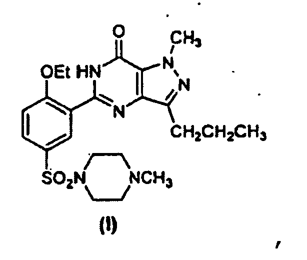 bei dem eine Verbindung ausgewählt aus der Gruppe bestehend aus den Verbindungen der Formeln **(II), (III)** und **(IV):** 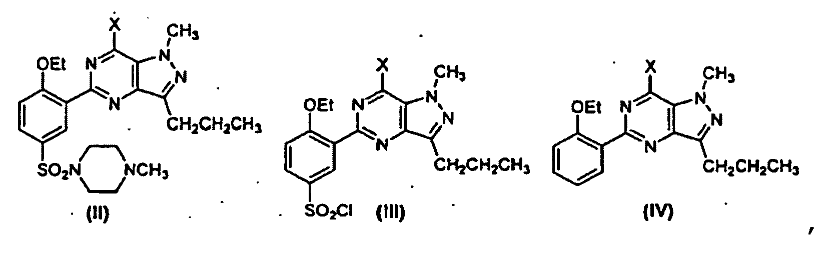 wobei X Halogen ist, in einem oder mehreren Schritten umgewandelt wird, um die Verbindung der Formel **(I)** zu ergeben.

2. Verfahren nach Anspruch 1, bei dem X Cl oder Br ist.

3. Verfahren nach Anspruch 1 oder 2, bei dem die Verbindung der Formel **(IV)** erhalten wird,
indem die Verbindung der Formel **(V)** in der Gegenwart von POX₃, PX₃, PX₅ oder Mischungen davon cyclisiert wird: 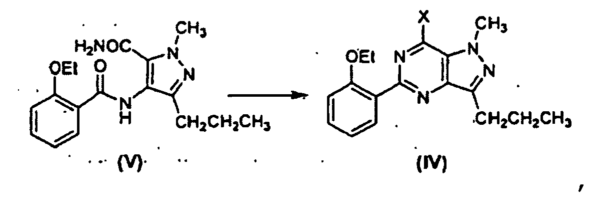 oder
indem die Verbindung der Formel **(X)** in der Gegenwart von POX₃, PX₃, PX₅ oder Mischungen halogeniert wird:

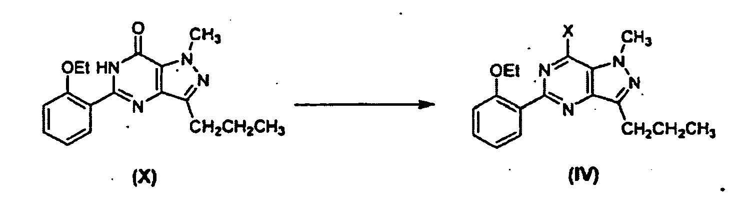

4. Verfahren nach Anspruch 3, bei dem die Verbindung der Formel **(IV)** erhalten wird,
indem die Verbindung der Formel **(V)** mit POX₃, PX₃, PX₅ oder Mischungen davon bei 50-120°C behandelt wird, oder
indem die Verbindung der Formel **(X)** mit POX₃, PX₃, PX₅ oder Mischungen davon bei 50-120°C behandelt wird.

5. Verfahren nach Anspruch 4, bei dem nach der Behandlung die Reaktionsmischung in Wasser, Eis oder Mischungen davon gegossen wird und die Ausfällung aufgefangen wird, um die Verbindung der Formel **(IV)** zu erhalten.

6. Verfahren nach einem der Ansprüche 3 bis 5, bei dem die Cyclisierungsreaktion oder die Halogenierungsreaktion in der Gegenwart von Benzol, Toluol, CH₂Cl₂, CHCl₃, ClCH₂CH₂Cl, THF, Dioxan oder Mischungen davon durchgeführt wird.

7. Verfahren nach einem der Ansprüche 1 bis 6, bei dem die Verbindung der Formel **(III)** erhalten wird, indem die Verbindung der Formel **(IV)** in der Gegenwart von Chlorsulfonsäure chlorsulfoniert wird:

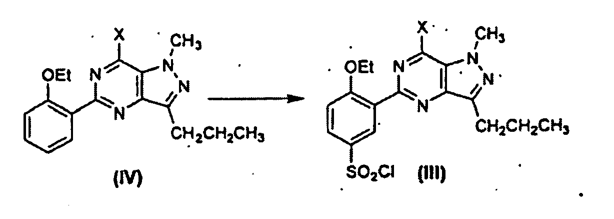

8. Verfahren nach einem der Ansprüche 1 bis 6, bei dem die Verbindung der Formel **(III)** erhalten wird, indem die Verbindung der Formel **(IV)** in der Gegenwart von POX₃, PX₃, PX₅ oder Mischungen davon cyclisiert wird und anschließend in Gegenwart von Chlorsulfonsäure ohne Reinigung des Zwischenproduktes chlorsulfoniert wird.

9. Verfahren nach Anspruch 1 oder 2, bei dem die Verbindung der Formel **(III)** erhalten wird,
indem die Verbindung der Formel **(VII)** in der Gegenwart von POX₃, PX₃, PX₅ oder Mischungen cyclisiert wird: 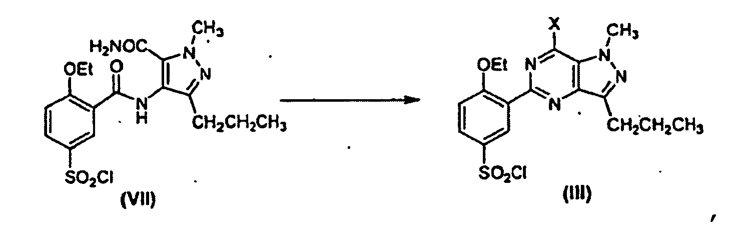 oder
indem die Verbindung der Formel **(VIII)** in der Gegenwart von POX₃, PX₃, PX₅ oder Mischungen halogeniert wird:

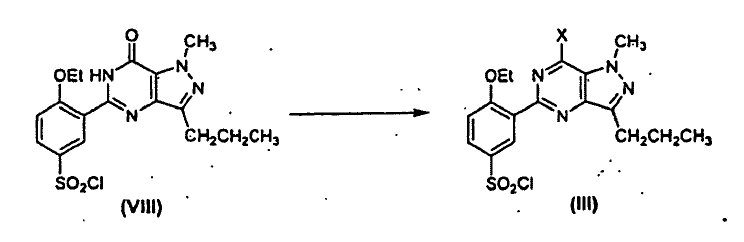

10. Verfahren nach Anspruch 7, bei dem die Verbindung der Formel **(III)** erhalten wird, indem die Verbindung der Formel **(IV)** mit Chlorsulfonsäure bei 0-50°C behandelt wird.

11. Verfahren nach Anspruch 9, bei dem die Verbindung der Formel **(III)** erhalten wird,
indem die Verbindung der Formel **(VII)** in der Gegenwart von POX₃, PX₃, PX₅ oder Mischungen davon auf 50-120°C erwärmt wird, oder
indem die Verbindung er Formel **(VIII)** in der Gegenwart von POX₃, PX₃, PX₅ oder Mischungen auf 50-120°C erwärmt wird.

12. Verfahren nach Anspruch 9 oder 11, bei dem die Cyclisierungsreaktion oder die Halogenierungsreaktion in der Gegenwart von Benzol, Toluol, CH₂Cl₂, CHCl₃, ClCH₂CH₂Cl, THF, Dioxan oder Mischungen davon durchgeführt wird.

13. Verfahren nach einem der Ansprüche 1 bis 12, bei dem die Verbindung der Formel **(II)** erhalten wird, indem die Verbindung der Formel **(III)** mit 1-Methylpiperazin behandelt wird:

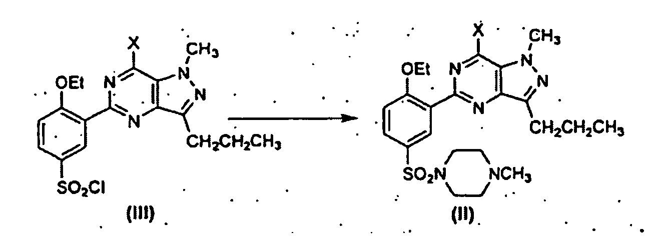

14. Verfahren nach Anspruch 1 oder 2, bei dem die Verbindung der Formel **(II)** erhalten wird, indem die Verbindung der Formel **(VI)** in der Gegenwart von POX₃, PX₃, PX₅ oder Mischungen davon cyclisiert wird.

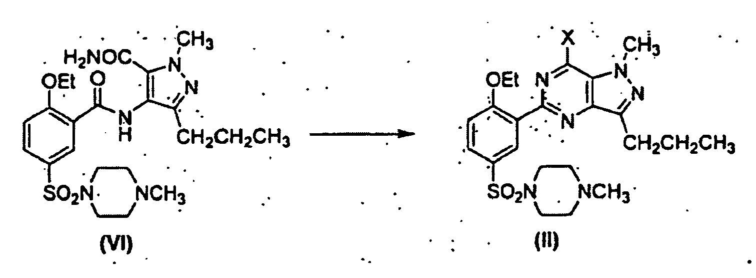

15. Verfahren nach Anspruch 13, bei dem die Verbindung der Formel **(II)** erhalten wird, indem die Verbindung der **(III)** mit 1-Methylpiperazin in der Gegenwart eines säureentfernenden Mittels behandelt wird.

16. Verfahren nach Anspruch 14, bei dem die Verbindung der Formel **(II)** erhalten wird, indem die Verbindung der Formel **(VI)** in der Gegenwart von POX₃, PX₃, PX₅ oder deren Mischungen in irgendeinem Verhältnis auf 50-120°C erwärmt wird.

17. Verfahren nach Anspruch 16, bei dem nach der Behandlung die Reaktionsmischung in Wasser, Eis oder Mischungen davon gegossen wird und die Ausfällung aufgefangen wird.

18. Verfahren nach Anspruch 16 oder 17, bei dem die Cyclisierung in Gegenwart von Benzol, Toluol, CH₂Cl₂, CHCl₃, ClCH₂CH₂Cl, THF, Dioxan oder deren Mischungen durchgeführt wird.

19. Verfahren nach einem der Ansprüche 1 bis 18, bei dem die Verbindung der Formel **(I)** erhalten wird, indem die Verbindung der Formel **(II)** umgesetzt wird, um die Verbindung der Formel **(I)** zu erhalten:

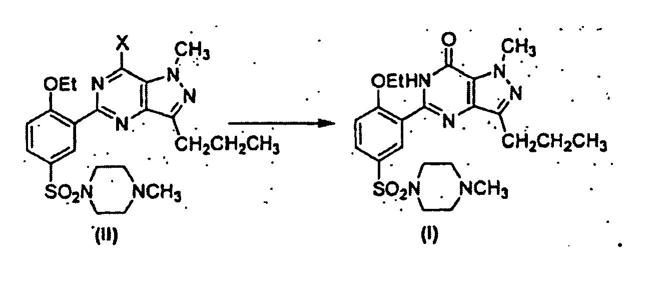

20. Verfahren nach Anspruch 19, bei dem die Reaktion in einem Lösungsmittel ausgewählt aus Wasser, Methanol, Ethanol, Isopropanol, t-C₄H₉OH, Glykol, Ethylenglykol-monomethylether und Mischungen davon durchgeführt wird.

21. Verfahren nach Anspruch 19 oder 20, bei dem die Reaktion in der Gegenwart einer Base oder Säure durchgeführt wird.

22. Verfahren nach Anspruch 21, bei dem die Base ausgewählt ist aus Alkalimetallalkoxiden, Alkalimetallhydriden, Erdalkalimetallhydriden, Aminen, Aminmetallderivaten, Hydroxiden, Carbonaten, Hydrogencarbonaten und Mischungen davon.

23. Verfahren nach Anspruch 21, bei dem die Säure ausgewählt ist aus Salzsäure, Schwefelsäure, Phosphorsäure, Zitronensäure, Weinsäure, Maleinsäure oder Mischungen davon.

24. Verfahren nach einem der Ansprüche 1 bis 23, bei dem außerdem die Verbindung der Formel **(I)** in ein pharmazeutisch annehmbares Salz oder Sulfat davon umgewandelt wird.

25. Verfahren nach Anspruch 24, bei dem das pharmazeutisch annehmbare Salz Sildenafilcitrat ist.

26. Verfahren nach Anspruch 25, bei dem die Umwandlung der Verbindung der Formel **(I)** zu Sildenafilcitrat erreicht wird, indem die Verbindung der Formel **(I)** mit Zitronensäure in einem geeigneten Lösungsmittel, vorzugsweise Methanol, in Kontakt gebracht wird.

27. Verfahren zur Herstellung einer pharmazeutischen Zusammensetzung, die die Verbindung der Formel **(I)** oder ein pharmazeutisch annehmbares Salz oder Solvat davon in Kombination mit mindestens einem pharmazeutisch annehmbaren Träger enthält, welches den Schritt der Herstellung der Verbindung der Formel **(I)** oder des pharmazeutisch annehmbaren Salzes oder Solvates davon gemäß dem Verfahren nach einem der Ansprüche 1 bis 26 aufweist.

28. Verbindung der Formel **(III)**: 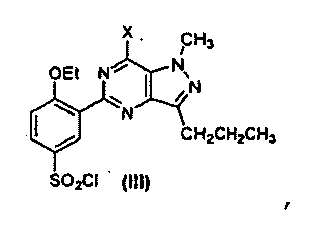 wobei X Cl oder Br ist.

## Revendications

1. Procédé de préparation du composé de formule (I) : 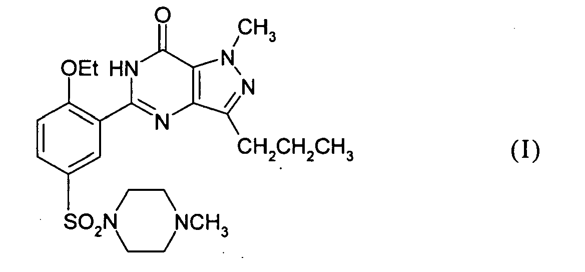 lequel procédé comporte le fait de convertir un composé, choisi dans l'ensemble formé par les composés de formules (II), (III) et (IV) : 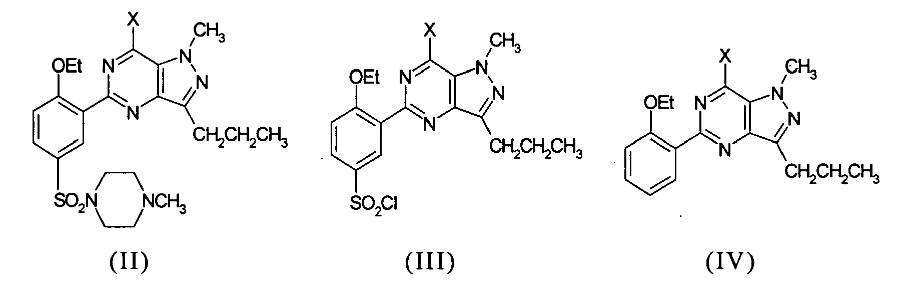 dans lesquelles X représente un atome d'halogène,
en une ou plusieurs étapes, pour obtenir le composé de formule (I).

2. Procédé conforme à la revendication 1, pour lequel X représente un atome de chlore ou de brome.

3. Procédé conforme à la revendication 1 ou 2, pour lequel on obtient le composé de formule (IV) :
soit par cyclisation du composé de formule (V) en présence d'un composé de formule POX₃, PX₃ ou PX₅ ou d'un mélange de tels composés : 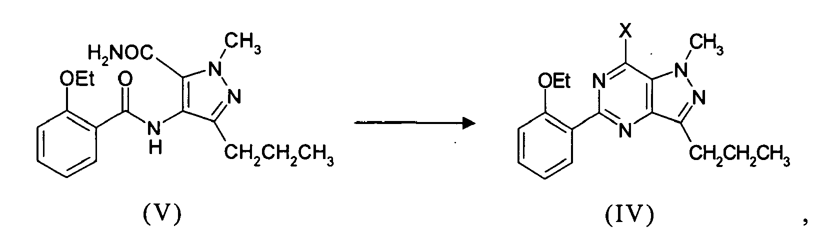 soit par halogénation du composé de formule (X) en présence d'un composé de formule POX₃, PX₃ ou PX₅ ou d'un mélange de tels composés :

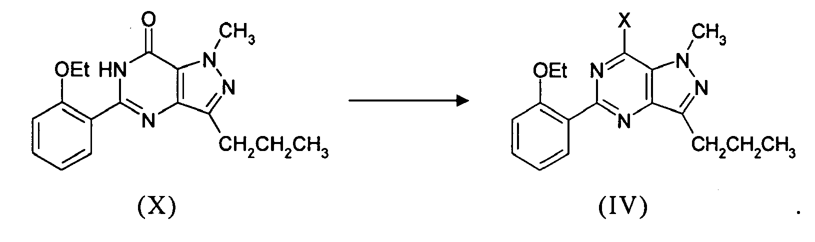

4. Procédé conforme à la revendication 3, pour lequel on obtient le composé de formule (IV) :
soit en traitant le composé de formule (V) avec un composé de formule POX₃, PX₃ ou PX₅ ou un mélange de tels composés, à une température de 50 à 120 °C,
soit en traitant le composé de formule (X) avec un composé de formule POX₃, PX₃ ou PX₅ ou un mélange de tels composés, à une température de 50 à 120 °C.

5. Procédé conforme à la revendication 4, dans lequel, à la suite dudit traitement, on verse le mélange réactionnel dans de l'eau, sur de la glace ou dans un mélange d'eau et de glace, et l'on recueille le précipité formé, pour obtenir le composé de formule (IV).

6. Procédé conforme à l'une des revendications 3 à 5, dans lequel on opère la réaction de cyclisation ou la réaction d'halogénation en présence de benzène, de toluène, de dichlorométhane, de trichlorométhane, de 1,2-dichloro-éthane, de tétrahydrofurane, de dioxane ou d'un mélange de ceux-ci.

7. Procédé conforme à l'une des revendications 1 à 6, pour lequel on obtient le composé de formule (III) par chlorosulfonation du composé de formule (IV) en présence d'acide chlorosulfonique :

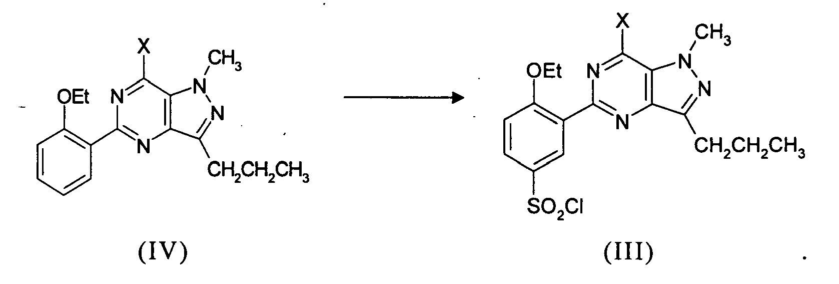

8. Procédé conforme à l'une des revendications 1 à 6, pour lequel on obtient le composé de formule (III) en opérant la cyclisation du composé de formule (V), en présence d'un composé de formule POX₃, PX₃ ou PX₅ ou d'un mélange de tels composés, puis, sans purifier le produit intermédiaire, la chlorosulfonation de celui-ci en présence d'acide chlorosulfonique.

9. Procédé conforme à la revendication 1 ou 2, pour lequel on obtient le composé de formule (III) :
soit par cyclisation du composé de formule (VII) en présence d'un composé de formule POX₃, PX₃ ou PX₅ ou d'un mélange de tels composés : 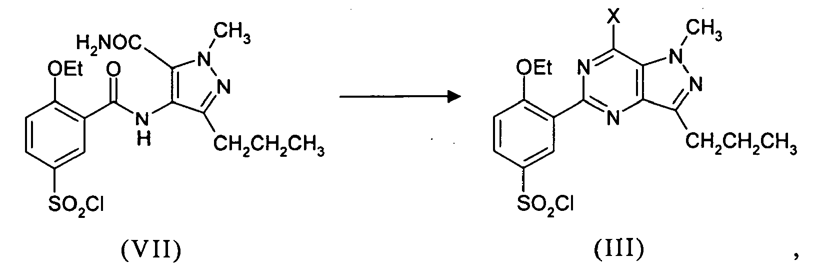 soit par halogénation du composé de formule (VIII) en présence d'un composé de formule POX₃, PX₃ ou PX₅ ou d'un mélange de tels composés :

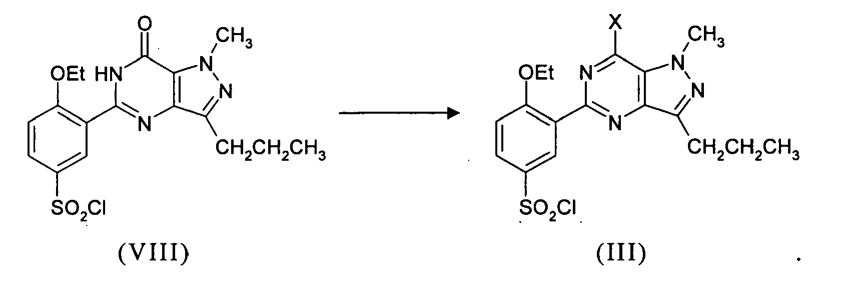

10. Procédé conforme à la revendication 7, dans lequel on obtient le composé de formule (III) en traitant le composé de formule (IV) avec de l'acide chlorosulfonique, à une température de 0 à 50 °C.

11. Procédé conforme à la revendication 9, dans lequel on obtient le composé de formule (III) :
soit en chauffant le composé de formule (VII) à une température de 50 à 120 °C, en présence d'un composé de formule POX₃, PX₃ ou PX₅ ou d'un mélange de tels composés,
soit en chauffant le composé de formule (VIII) à une température de 50 à 120 °C, en présence d'un composé de formule POX₃, PX₃ ou PX₅ ou d'un mélange de tels composés.

12. Procédé conforme à la revendication 9 ou 11, dans lequel on opère la réaction de cyclisation ou la réaction d'halogénation en présence de benzène, de toluène, de dichlorométhane, de trichlorométhane, de 1,2-dichloro-éthane, de tétrahydrofurane, de dioxane ou d'un mélange de ceux-ci.

13. Procédé conforme à l'une des revendications 1 à 12, pour lequel on obtient le composé de formule (II) en traitant le composé de formule (III) avec de la 1-méthyl-pipérazine :

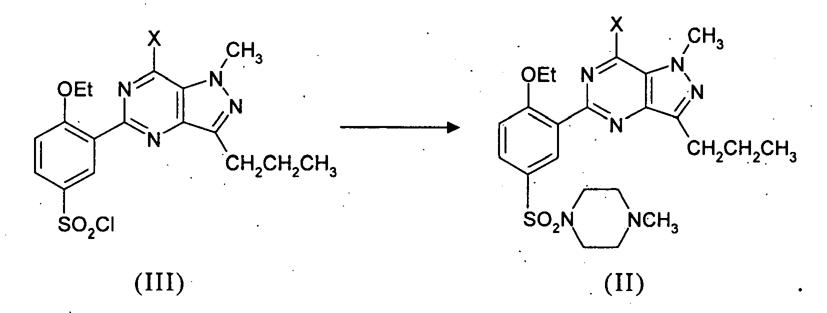

14. Procédé conforme à la revendication 1 ou 2, pour lequel on obtient le composé de formule (II) par cyclisation du composé de formule (VI) en présence d'un composé de formule POX₃, PX₃ ou PX₅ ou d'un mélange de tels composés :

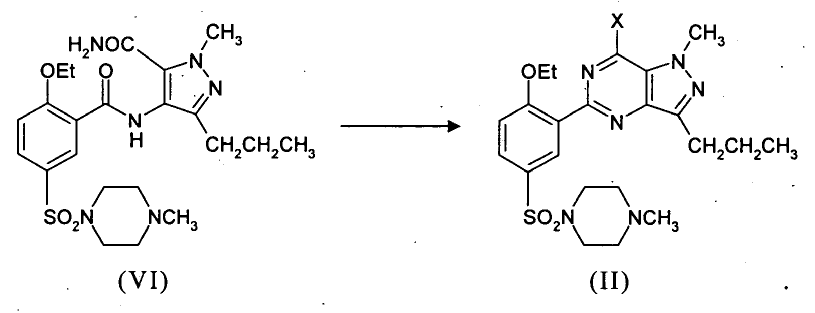

15. Procédé conforme à la revendication 13, dans lequel on obtient le composé de formule (II) en traitant le composé de formule (III) avec de la 1-méthyl-pipérazine, en présence d'un agent d'élimination d'acide.

16. Procédé conforme à la revendication 14, dans lequel on obtient le composé de formule (II) en chauffant le composé de formule (VI) à une température de 50 à 120 °C, en présence d'un composé de formule POX₃, PX₃ ou PX₅ ou d'un mélange en n'importe quelles proportions de tels composés.

17. Procédé conforme à la revendication 16, dans lequel, à la suite dudit traitement, on verse le mélange réactionnel dans de l'eau, sur de la glace ou dans un mélange d'eau et de glace, et l'on recueille le précipité formé.

18. Procédé conforme à la revendication 16 ou 17, dans lequel on opère la réaction de cyclisation en présence de benzène, de toluène, de dichlorométhane, de trichlorométhane, de 1,2-dichloro-éthane, de tétrahydrofurane, de dioxane ou d'un mélange de ceux-ci.

19. Procédé conforme à l'une des revendications 1 à 18, dans lequel on obtient le composé de formule (I) en faisant réagir le composé de formule (II) de manière à obtenir le composé de formule (I) :

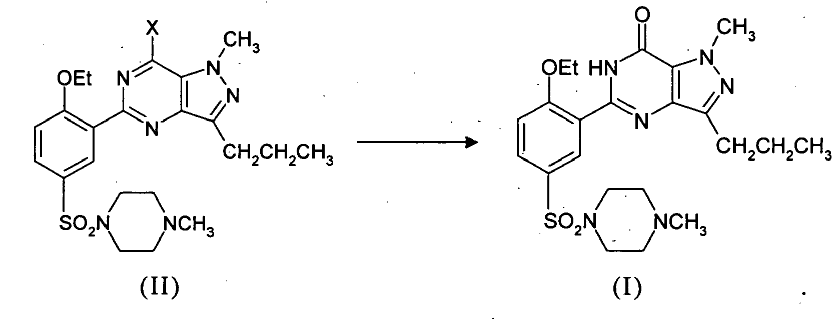

20. Procédé conforme à la revendication 19, dans lequel on effectue la réaction dans un solvant choisi parmi l'eau, les méthanol, éthanol, isopropanol, tertiobutanol, glycol et éther monométhylique d'éthylèneglycol, et les mélanges de ces solvants.

21. Procédé conforme à la revendication 19 ou 20, dans lequel on effectue la réaction en présence d'une base ou d'un acide.

22. Procédé conforme à la revendication 21, dans lequel la base est choisie parmi les alcoolates de métal alcalin, hydrures de métal alcalin, hydrures de métal alcalino-terreux, amines, dérivés métallés d'amine, hydroxydes, carbonates et bicarbonates, et les mélanges de tels composés.

23. Procédé conforme à la revendication 21, dans lequel l'acide est choisi parmi l'acide chlorhydrique, l'acide sulfurique, l'acide phosphorique, l'acide citrique, l'acide tartrique, l'acide maléique et leurs mélanges.

24. Procédé conforme à l'une des revendications 1 à 23, qui comporte en outre le fait de convertir le composé de formule (I) en l'un de ses sels ou solvats pharmacologiquement admissibles.

25. Procédé conforme à la revendication 24, dans lequel le sel pharmacologiquement admissible est le citrate de sildénafil.

26. Procédé conforme à la revendication 25, dans lequel on opère la conversion du composé de formule (I) en citrate de sildénafil en mettant le composé de formule (I) en contact avec de l'acide citrique, dans un solvant approprié, de préférence dans du méthanol.

27. Procédé de préparation d'une composition pharmaceutique comprenant du composé de formule (I), ou de l'un de ses sels ou solvats pharmacologiquement admissibles, en combinaison avec au moins un véhicule pharmacologiquement admissible, lequel procédé comporte le fait de préparer le composé de formule (I), ou l'un de ses sels ou solvats pharmacologiquement admissibles, en opérant selon un procédé conforme à l'une des revendications 1 à 26.

28. Composé de formule (III) : 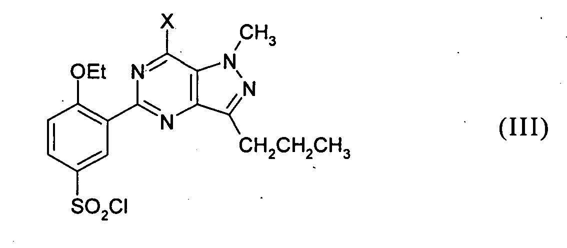 dans laquelle X représente un atome de chlore ou de brome.
